# EUROPEAN PATENT APPLICATION

(11) **EP 4 289 836 A1**
(43) Date of publication of application: **13.12.2023**
(21) Application number: 22853382.4
(22) Date of filing: 01.08.2022
(51) Int. Cl.: C07D 405/04, H10K 99/00, H10K 50/00

(54) **NOVEL COMPOUND AND ORGANIC LIGHT-EMITTING DEVICE COMPRISING SAME**

(30) Priority: 02.08.2021 KR 20210101385
(71) Applicant: Lg Chem, Ltd., Seoul 07336 (KR)
(72) Inventor: SUH, Sang Duk, Daejeon 34122 (KR); KIM, Minjun, Daejeon 34122 (KR); KIM, Young Seok, Daejeon 34122 (KR); KIM, Donghee, Daejeon 34122 (KR); OH, Joongsuk, Daejeon 34122 (KR); LEE, Da Jung, Daejeon 34122 (KR); LEE, Dong Hoon, Daejeon 34122 (KR)
(74) Representative: Hoffmann Eitle
(86) International application number: PCT/KR2022/011266
(87) International publication number: WO 2023/014004

(57) **Abstract**

The present disclosure provides a novel compound and an organic light emitting device including the same.

## Description

### [TECHNICAL FIELD]

### CROSS-REFERENCE TO RELATED APPLICATION(S)

This application claims the benefit of Korean Patent Application No. 10-2021-0101385 filed on August 2, 2021 with the Korean Intellectual Property Office, the content of which is incorporated herein by reference in its entirety.

The present disclosure relates to a novel compound and an organic light emitting device comprising the same.

### [BACKGROUND ART]

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently has a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer, and the like. In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

There is a continuing need for the development of a new material for an organic material used in the organic light emitting device as described above.

### [Prior Art Literature]

### [Patent Literature]

(Patent Literature 0001) Korean Unexamined Patent Publication No. 10-2000-0051826

### [DETAILED DESCRIPTION OF THE INVENTION]

### [Technical Problem]

It is an object of the present disclosure to provide a novel compound and an organic light emitting device comprising the same.

### [Technical Solution]

According to an aspect of the present disclosure, there is provided a compound represented by the following Chemical Formula 1: 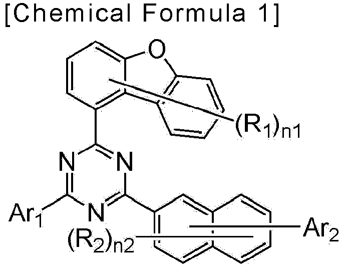 in Chemical Formula 1,
Ar₁ and Ar₂ are each independently a substituted or unsubstituted C₆₋₁₅ aryl,
R₁ and R₂ are each independently hydrogen or deuterium,
n1 is an integer of 1 to 7, and
n2 is an integer of 1 to 6.

According to another aspect of the present disclosure, there is provided an organic light emitting device comprising: a first electrode; a second electrode that is provided opposite to the first electrode; and one or more organic material layers that are provided between the first electrode and the second electrode, wherein the one or more organic material layers comprise the compound represented by Chemical Formula 1.

### [ADVANTAGEOUS EFFECTS]

The above-mentioned compound represented by Chemical Formula 1 can be used as a material for an organic material layer of an organic light emitting device, and can improve the efficiency, achieve low driving voltage and/or improve lifetime characteristics in the organic light emitting device. In particular, the compound represented by Chemical Formula 1 described above can be used as a material for hole injection, hole transport, light emission, electron transport and injection.

### [BRIEF DESCRIPTION OF THE DRAWINGS]

FIG. 1 shows an example of an organic light emitting device comprising a substrate 1, an anode 2, a light emitting layer 3, and a cathode 4.
FIG. 2 shows an example of an organic light emitting device comprising a substrate 1, an anode 2, a hole injection layer 5, a hole transport layer 6, an electron blocking layer 7, a light emitting layer 3, an electron injection and transport layer 8, and a cathode 4.

### [DETAILED DESCRIPTION OF THE EMBODIMENTS]

Hereinafter, embodiments of the present disclosure will be described in more detail to facilitate understanding of the invention.

The present disclosure provides the compound represented by Chemical Formula 1.

As used herein, the notation 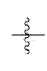 and 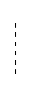 mean a bond linked to another substituent group.

As used herein, the term "substituted or unsubstituted" means being unsubstituted or substituted with one or more substituents selected from the group consisting of deuterium; a halogen group; a nitrile group; a nitro group; a hydroxy group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; and a heteroaryl containing at least one of N, O and S atoms, or being unsubstituted or substituted with a substituent to which two or more substituents of the above-exemplified substituents are linked. For example, "a substituent in which two or more substituents are linked" may be a biphenyl group. Namely, a biphenyl group may be an aryl group, or it may also be interpreted as a substituent in which two phenyl groups are linked.

In the present disclosure, the carbon number of a carbonyl group is not particularly limited, but is preferably 1 to 40. Specifically, the carbonyl group may be a substituent group having the following structural formulas, but is not limited thereto.

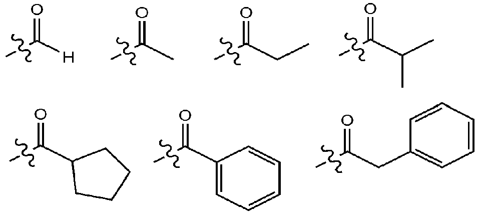

In the present disclosure, an ester group may have a structure in which oxygen of the ester group may be substituted by a straight-chain, branched-chain, or cyclic alkyl group having 1 to 25 carbon atoms, or an aryl group having 6 to 25 carbon atoms. Specifically, the ester group may be a substituent group having the following structural formulas, but is not limited thereto.

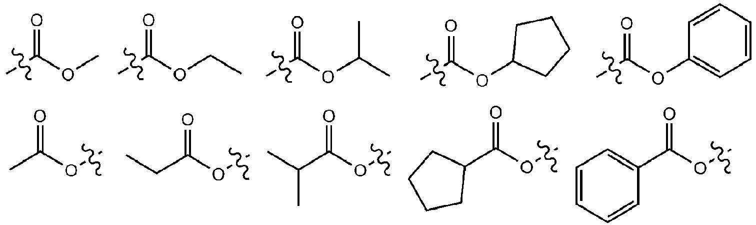

In the present disclosure, the carbon number of an imide group is not particularly limited, but is preferably 1 to 25. Specifically, the imide group may be a substituent group having the following structural formulas, but is not limited thereto.

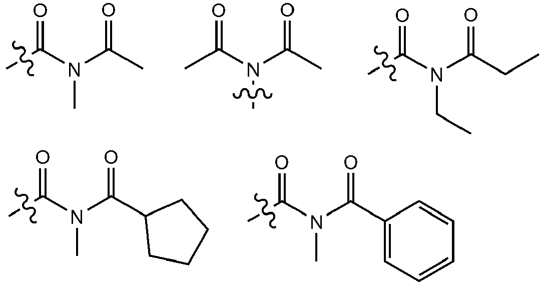

In the present disclosure, a silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but is not limited thereto.

In the present disclosure, a boron group specifically includes a trimethylboron group, a triethylboron group, a t-butyldimethylboron group, a triphenylboron group, and a phenylboron group, but is not limited thereto.

In the present disclosure, examples of a halogen group include fluorine, chlorine, bromine, or iodine.

In the present disclosure, the alkyl group may be straight-chain or branched-chain, and the carbon number thereof is not particularly limited, but is preferably 1 to 40. According to one embodiment, the carbon number of the alkyl group is 1 to 20. According to another embodiment, the carbon number of the alkyl group is 1 to 10. According to another embodiment, the carbon number of the alkyl group is 1 to 6. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohectylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl, 5-methylhexyl, and the like, but are not limited thereto.

In the present disclosure, the alkenyl group may be straight-chain or branched-chain, and the carbon number thereof is not particularly limited, but is preferably 2 to 40. According to one embodiment, the carbon number of the alkenyl group is 2 to 20. According to another embodiment, the carbon number of the alkenyl group is 2 to 10. According to still another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present disclosure, a cycloalkyl group is not particularly limited, but the carbon number thereof is preferably 3 to 60. According to one embodiment, the carbon number of the cycloalkyl group is 3 to 30. According to another embodiment, the carbon number of the cycloalkyl group is 3 to 20. According to still another embodiment, the carbon number of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present disclosure, an aryl group is not particularly limited, but the carbon number thereof is preferably 6 to 60, and it may be a monocyclic aryl group or a polycyclic aryl group. According to one embodiment, the carbon number of the aryl group is 6 to 30. According to one embodiment, the carbon number of the aryl group is 6 to 20. The aryl group may be a phenyl group, a biphenyl group, a terphenyl group or the like as the monocyclic aryl group, but is not limited thereto. The polycyclic aryl group includes a naphthyl group, an anthracenyl group, a phenanthryl group, a pyrenyl group, a perylenyl group, a chrysenyl group, a fluorenyl group, or the like, but is not limited thereto.

In the present disclosure, the fluorenyl group may be substituted, and two substituents may be connected to each other to form a spiro structure. In the case where the fluorenyl group is substituted, 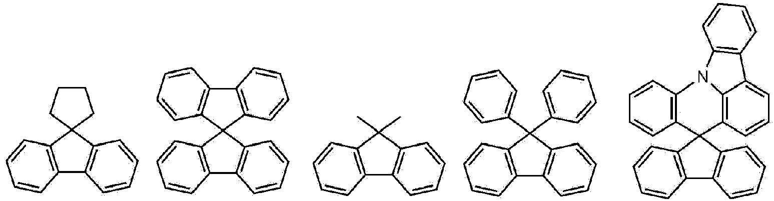 and the like can be formed. However, the structure is not limited thereto

In the present disclosure, a heteroaryl group is a heteroaryl group containing at least one of O, N, Si and S as a heteroatom, and the carbon number thereof is not particularly limited, but is preferably 2 to 60. According to an embodiment, the carbon number of the heteroaryl group is 6 to 30. According to an embodiment, the carbon number of the heteroaryl group is 6 to 20. Examples of heteroaryl groups include a thiophene group, a furan group, a pyrrole group, an imidazole group, a thiazole group, an oxazol group, an oxadiazol group, a triazol group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzoimidazole group, a benzothiazol group, a benzocarbazole group, a benzothiophene group, a dibenzothiophene group, a benzofuranyl group, a phenanthroline group, an isoxazolyl group, a thiadiazolyl group, a phenothiazinyl group, a dibenzofuranyl group, and the like, but are not limited thereto.

In the present disclosure, the aryl group in the aralkyl group, the aralkenyl group, the alkylaryl group and the arylamine group is the same as the above-mentioned examples of the aryl group. In the present disclosure, the alkyl group in the aralkyl group, the alkylaryl group and the alkylamine group is the same as the above-mentioned examples of the alkyl group. In the present disclosure, the heteroaryl in the heteroarylamine may be applied to the above-mentioned description of the heteroaryl group. In the present disclosure, the alkenyl group in the aralkenyl group is the same as the above-mentioned examples of the alkenyl group. In the present disclosure, the above-mentioned description of the aryl group may be applied except that the arylene is a divalent group. In the present disclosure, the above-mentioned description of the heteroaryl group may be applied except that the heteroarylene is a divalent group. In the present disclosure, the above-mentioned description of the aryl group or cycloalkyl group may be applied except that the hydrocarbon ring is not a monovalent group but formed by combining two substituent groups. In the present disclosure, the above-mentioned description of the heteroaryl group may be applied, except that the heteroaryl is not a monovalent group but formed by combining two substituent groups.

Preferably, the Chemical Formula 1 is represented by any one of the following Chemical Formulas 1-1 to 1-6 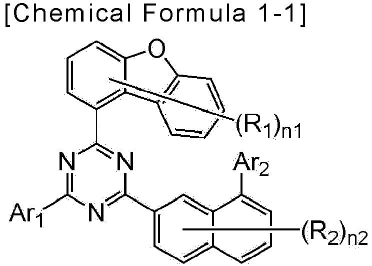 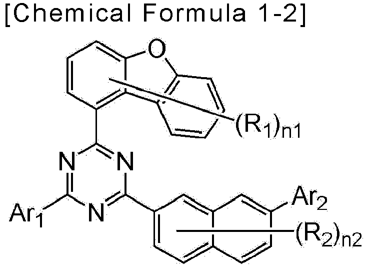 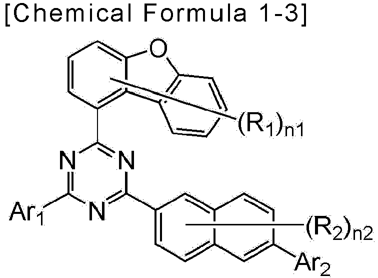 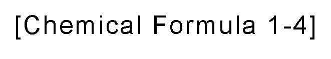 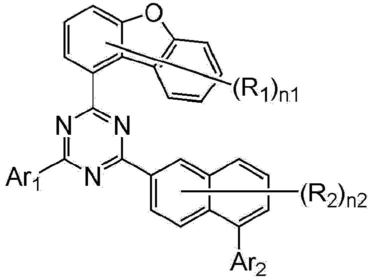 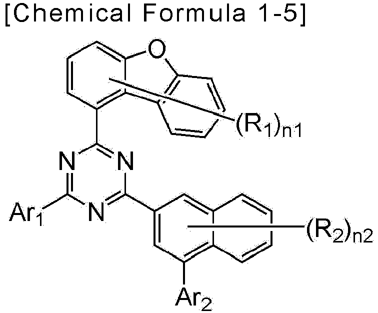 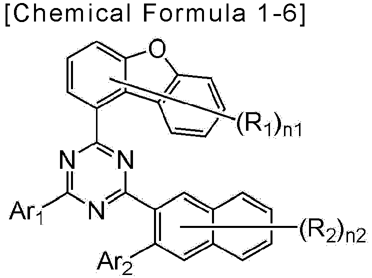 in Chemical Formulas 1-1 to 1-6,
Ar₁, Ar₂, R₁, R₂, n1 and n2 are as defined in Chemical Formula 1.

Preferably, Ar₁ and Ar₂ are each independently a C₆₋₁₄ aryl which is unsubstituted or substituted with deuterium. More preferably, Ar₁ and Ar₂ are each independently phenyl, phenyl substituted with 3 or 5 deuteriums, biphenylyl, biphenylyl substituted with 5 or 9 deuteriums, naphthyl, naphthyl substituted with 7 deuteriums, phenanthrenyl, or phenanthrenyl substituted with 9 deuteriums. Most preferably, Ar₁ and Ar₂ may be each independently any one selected from the group consisting of:

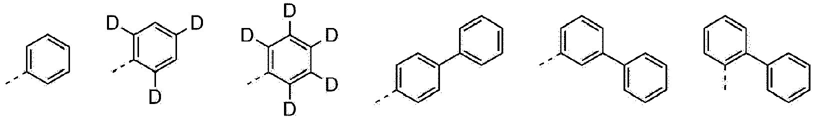

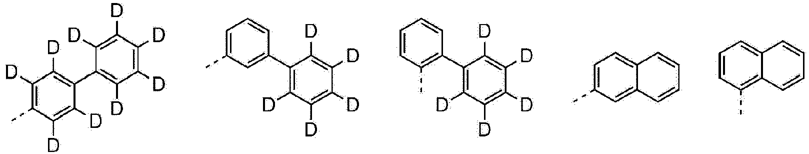

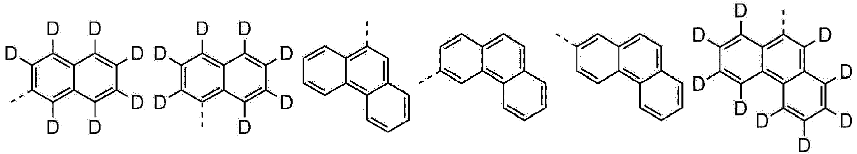

Preferably, Ar₁ may be any one selected from the group consisting of:

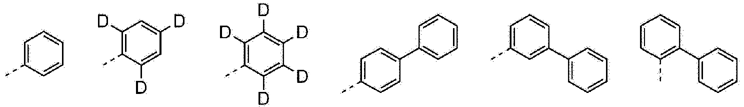

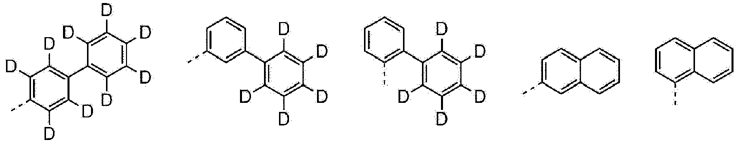

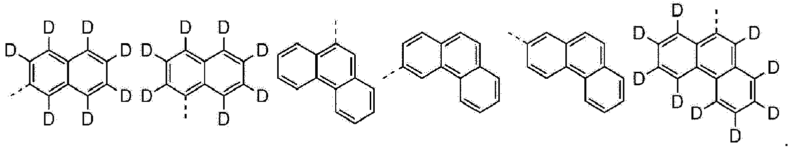

Preferably, each Ar₂ is independently phenyl, phenyl substituted with 5 deuterium, biphenylyl, biphenylyl substituted with 5 or 9 deuteriums, naphthyl, naphthyl substituted with 7 deuteriums, or phenanthrenyl. More preferably, Ar₂ may be any one selected from the group consisting of:

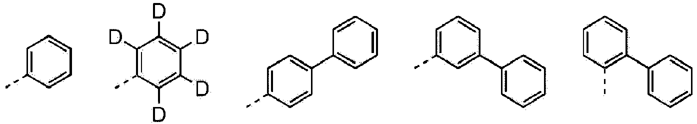

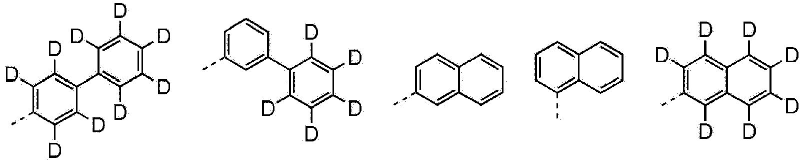

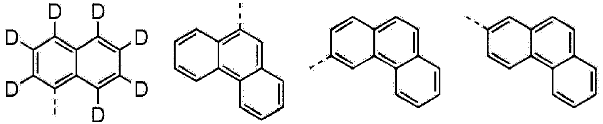

Preferably, at least one of Ar₁ and Ar₂ may be phenyl or phenyl substituted with 3 or 5 deuteriums.

Representative examples of the compound represented by Chemical Formula 1 are as follows:

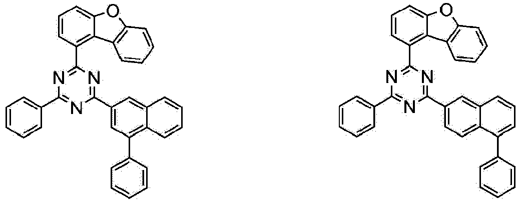

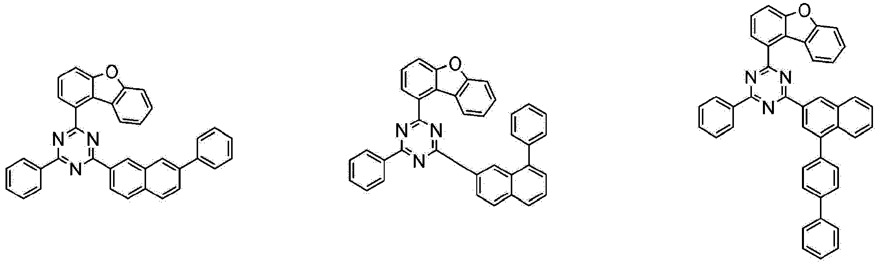

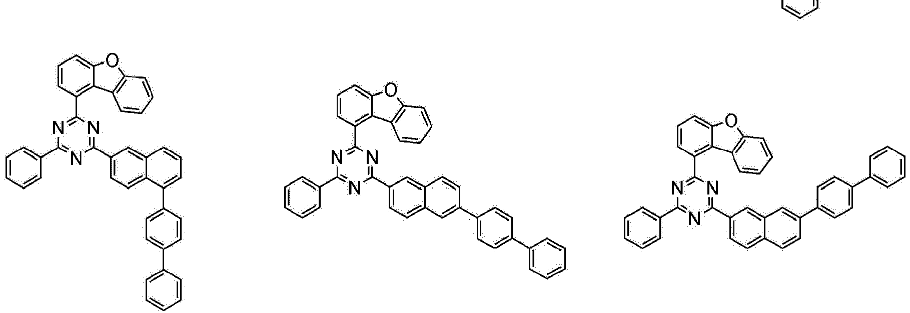

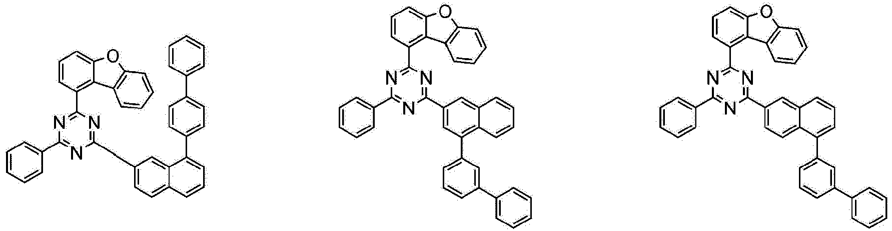

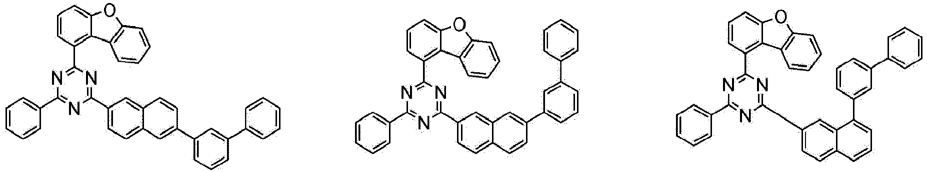

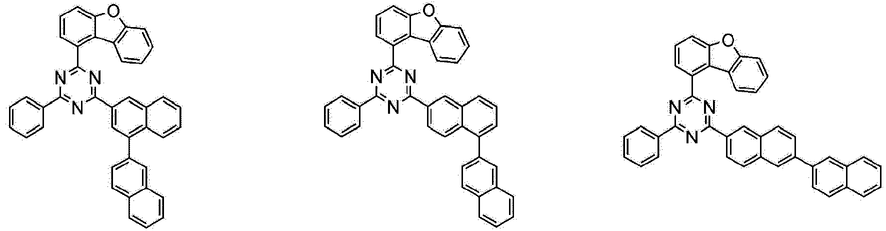

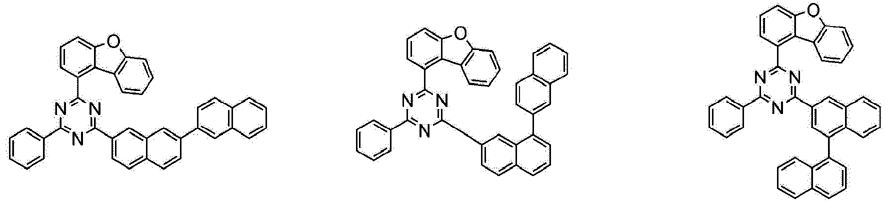

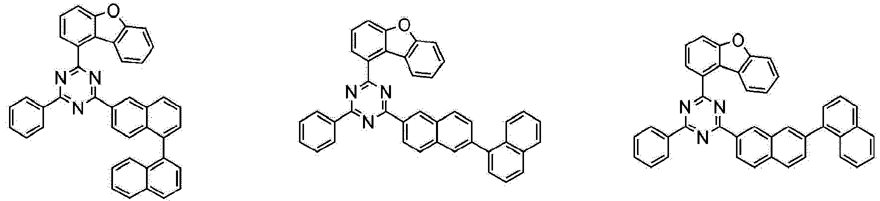

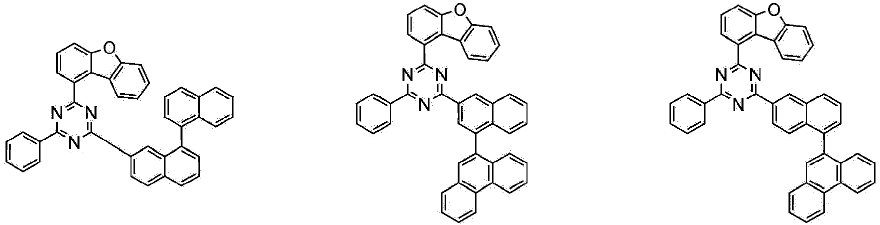

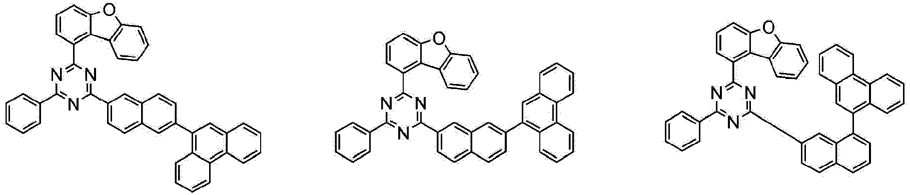

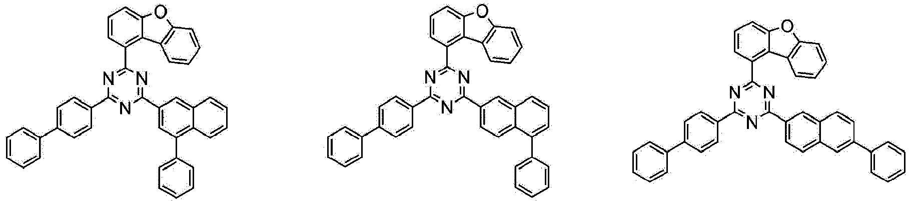

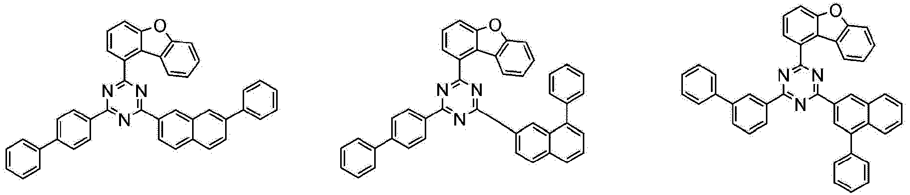

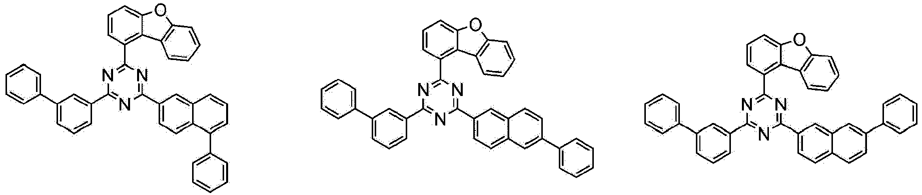

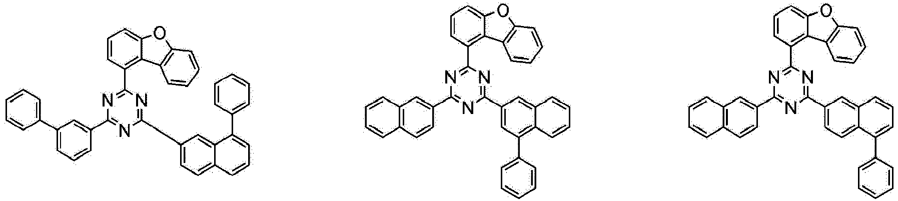

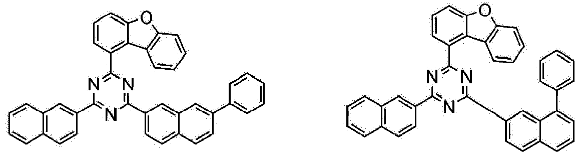

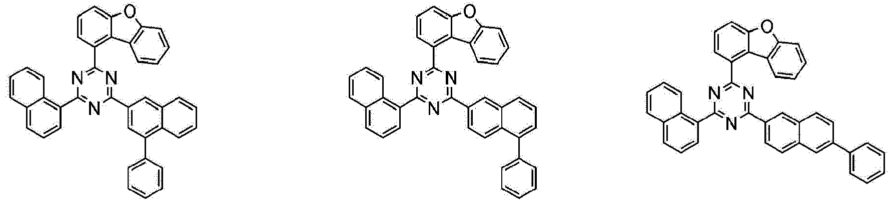

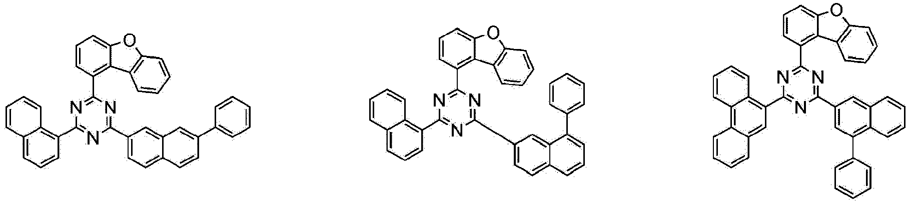

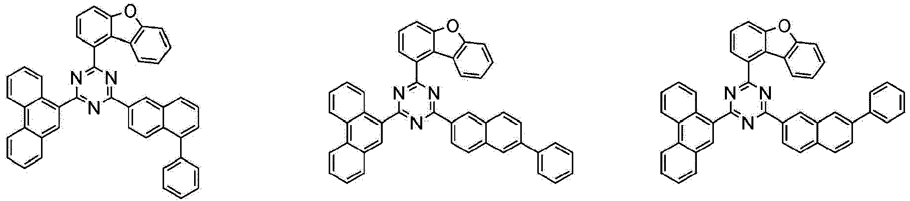

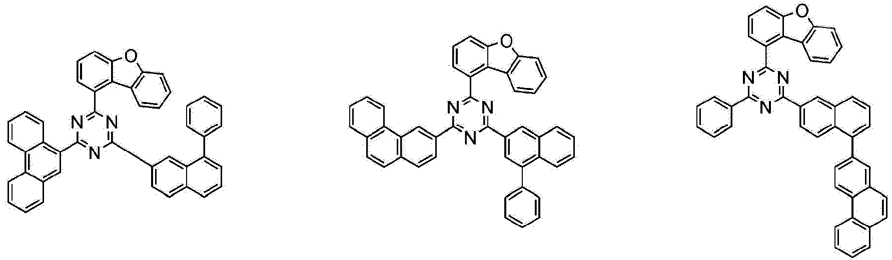

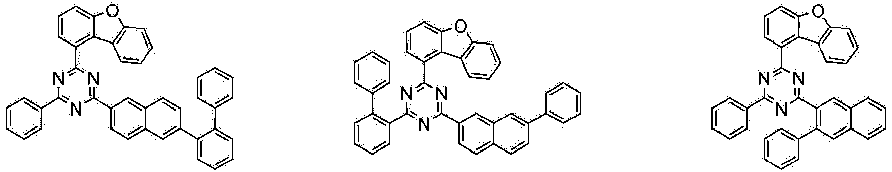

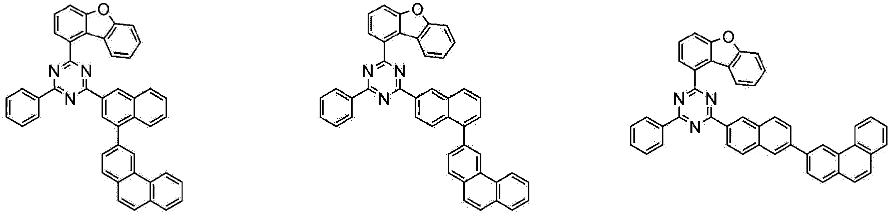

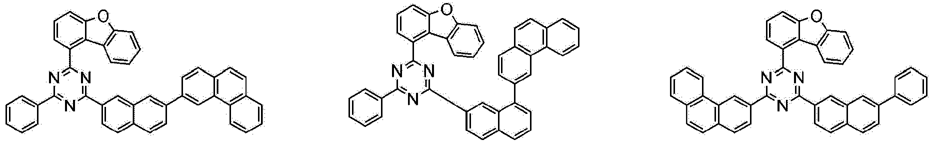

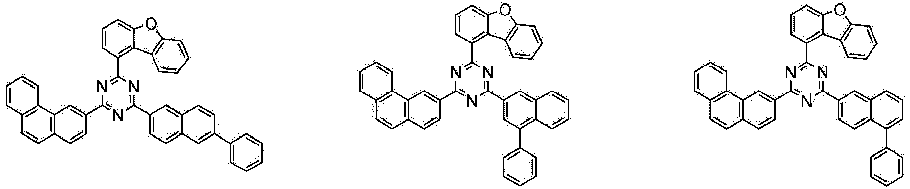

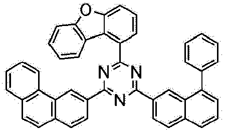

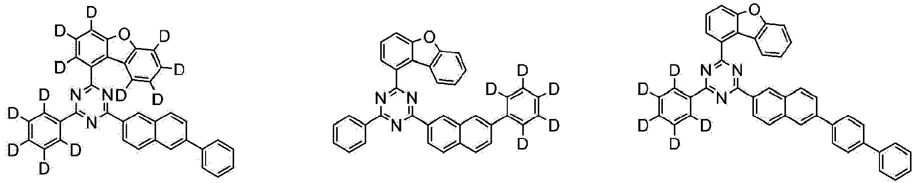

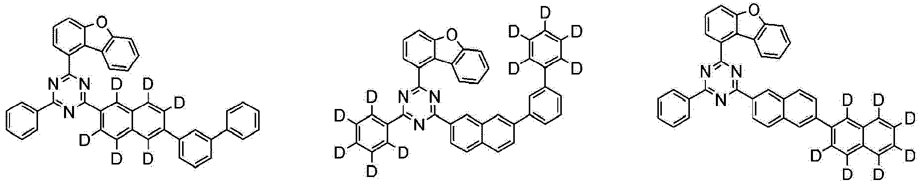

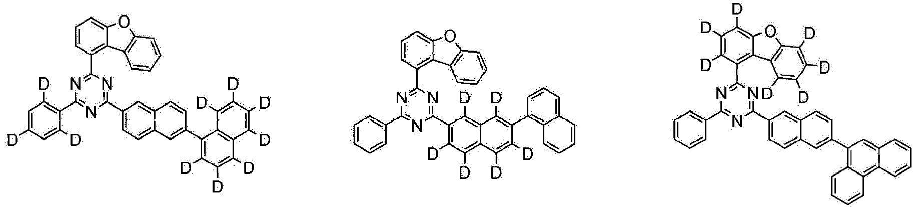

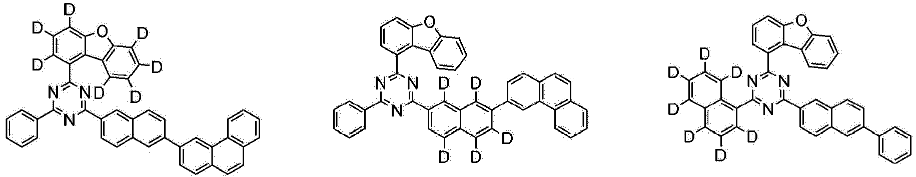

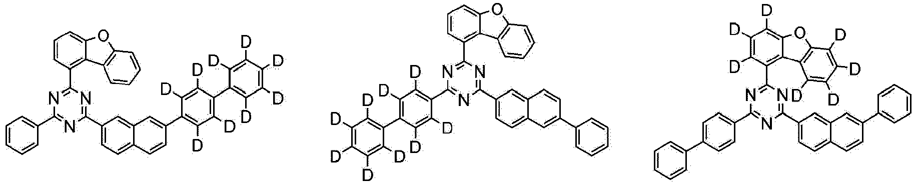

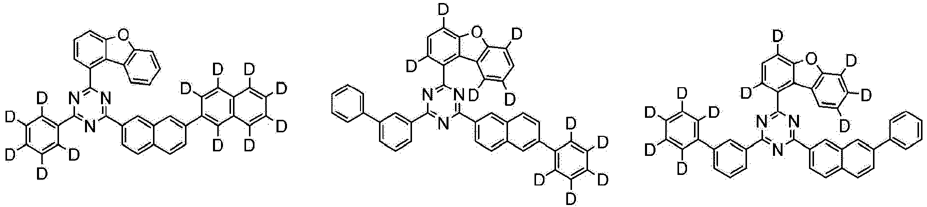

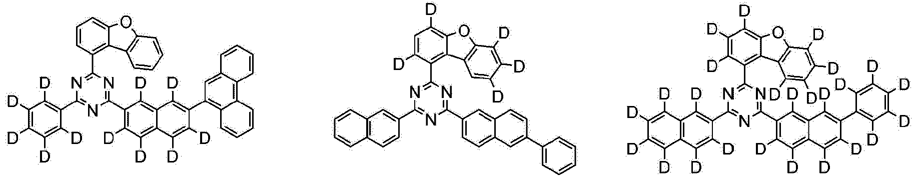

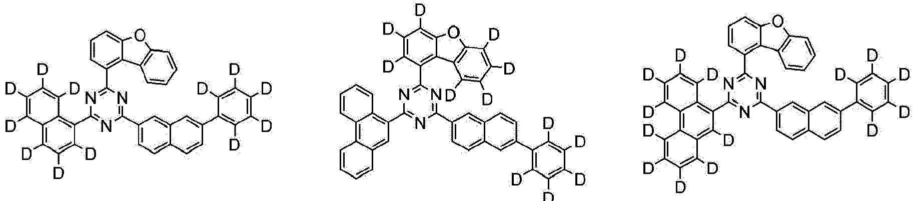

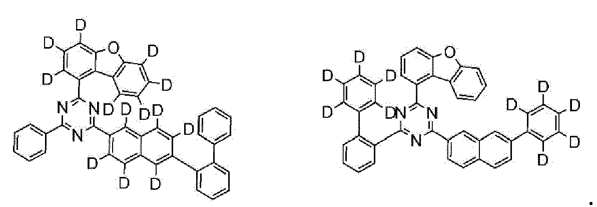

The compound represented by Chemical Formula 1 can be prepared by a preparation method as shown in the following Reaction Scheme 1 as an example, and other remaining compounds can be prepared in a similar manner.

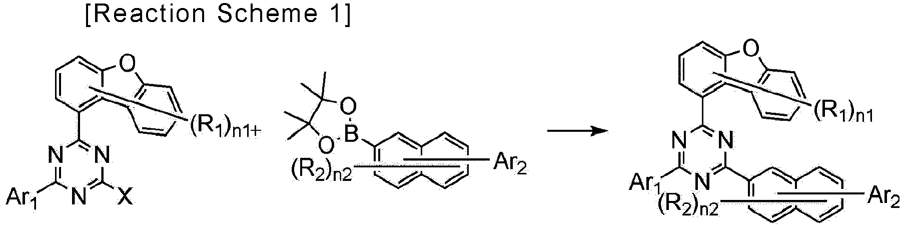

In Reaction Scheme 1, Ar₁, Ar₂, R₁, R₂, n1 and n2 are as defined in Chemical Formula 1, X is halogen, and preferably X is chloro or bromo.

Reaction Scheme 1 is a Suzuki coupling reaction, which is preferably carried out in the presence of a palladium catalyst and a base, and a reactive group for the Suzuki coupling reaction can be modified as known in the art. The above preparation method may be further embodied in Preparation Examples described hereinafter.

Further, the present disclosure provides an organic light emitting device including the compound represented by Chemical Formula 1. In one example, the present disclosure provides an organic light emitting device comprising: a first electrode; a second electrode that is provided opposite to the first electrode; and one or more organic material layers that are provided between the first electrode and the second electrode, wherein the one or more organic material layers include the compound represented by Chemical Formula 1.

The organic material layer of the organic light emitting device of the present disclosure may have a single-layer structure, or it may have a multilayered structure in which two or more organic material layers are stacked. For example, the organic light emitting device of the present disclosure may have a structure comprising a hole injection layer, a hole transport layer, a light emitting layer, an electron transport layer, an electron injection layer and the like as the organic material layer. However, the structure of the organic light emitting device is not limited thereto, and it may include a smaller number of organic material layers.

Further, the organic material layer may include a light emitting layer, wherein the light emitting layer may include the compound represented by Chemical Formula 1.

Further, the organic material layer may include a hole transport layer, a hole injection layer, or a layer that simultaneously performs hole transport and hole injection, wherein the hole transport layer, the hole injection layer, or the layer that simultaneously performs hole transport and hole injection may include the compound represented by Chemical Formula 1.

Further, the organic material layer may include an electron transport layer, an electron injection layer, or an electron injection and transport layer, wherein the electron transport layer, the electron injection layer, or the electron injection and transport layer may include the compound represented by Chemical Formula 1.

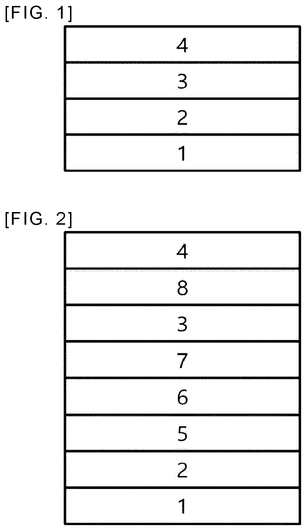

Further, the organic light emitting device according to the present disclosure may be a normal type organic light emitting device in which an anode, one or more organic material layers, and a cathode are sequentially stacked on a substrate. Further, the organic light emitting device according to the present disclosure may be an inverted type organic light emitting device in which a cathode, one or more organic material layers, and an anode are sequentially stacked on a substrate. For example, the structure of the organic light emitting device according to an embodiment of the present disclosure is illustrated in Figs. 1 and 2.

FIG. 1 shows an example of an organic light emitting device comprising a substrate 1, an anode 2, a light emitting layer 3, and a cathode 4. FIG. 2 shows an example of an organic light emitting device comprising a substrate 1, an anode 2, a hole injection layer 5, a hole transport layer 6, an electron blocking layer 7, a light emitting layer 3, an electron injection and transport layer 8, and a cathode 4. In such a structure, the compound represented by Chemical Formula 1 may be included in the light emitting layer.

The organic light emitting device according to the present disclosure may be manufactured by materials and methods known in the art, except that at least one of the organic material layers includes the compound represented by Chemical Formula 1. Further, when the organic light emitting device includes a plurality of organic material layers, the organic material layers may be formed of the same material or different materials.

For example, the organic light emitting device according to the present disclosure can be manufactured by sequentially stacking a first electrode, an organic material layer and a second electrode on a substrate. In this case, the organic light emitting device may be manufactured by depositing a metal, metal oxides having conductivity, or an alloy thereof on the substrate using a PVD (physical vapor deposition) method such as a sputtering method or an e-beam evaporation method to form an anode, forming organic material layers including the hole injection layer, the hole transport layer, the light emitting layer and the electron transport layer thereon, and then depositing a material that can be used as the cathode thereon. In addition to such a method, the organic light emitting device can be manufactured by sequentially depositing a cathode material, an organic material layer and an anode material on a substrate.

Further, the compound represented by Chemical Formula 1 can be formed into an organic material layer by a solution coating method as well as a vacuum deposition method at the time of manufacturing an organic light emitting device. Wherein, the solution coating method means a spin coating, a dip coating, a doctor blading, an inkjet printing, a screen printing, a spray method, a roll coating, or the like, but is not limited thereto.

In addition to such a method, the organic light emitting device may be manufactured by sequentially depositing a cathode material, an organic material layer and an anode material on a substrate (International Publication WO2003/012890). However, the manufacturing method is not limited thereto.

In one example, the first electrode is an anode, and the second electrode is a cathode, or alternatively, the first electrode is a cathode and the second electrode is an anode.

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:Al or SnO₂:Sb; conductive polymers such as poly(3-methylthiophene), poly[3,4-(ethylene-1,2-dioxy)thiophene](PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or LiO₂/Al, and the like, but are not limited thereto.

The hole injection layer is a layer for injecting holes from the electrode, and the hole injection material is preferably a compound which has a capability of transporting the holes, thus has a hole injecting effect in the anode and an excellent hole injecting effect to the light emitting layer or the light emitting material, prevents excitons produced in the light emitting layer from moving to an electron injection layer or the electron injection material, and further is excellent in the ability to form a thin film. It is preferable that a HOMO (highest occupied molecular orbital) of the hole injection material is between the work function of the anode material and a HOMO of a peripheral organic material layer. Specific examples of the hole injection material include metal porphyrin, oligothiophene, an arylamine-based organic material, a hexanitrilehexaazatriphenylene-based organic material, a quinacridone-based organic material, a perylene-based organic material, anthraquinone, polyaniline and polythiophene-based conductive polymer, and the like, but are not limited thereto.

The hole transport layer is a layer that receives holes from a hole injection layer and transports the holes to the light emitting layer. The hole transport material is suitably a material having large mobility to the holes, which may receive holes from the anode or the hole injection layer and transfer the holes to the light emitting layer. Specific examples thereof include an arylamine-based organic material, a conductive polymer, a block copolymer in which a conjugate portion and a non-conjugate portion are present together, and the like, but are not limited thereto.

The electron blocking layer is a layer provided between the hole transport layer and the light emitting layer in order to prevent the electrons injected in the cathode from being transferred to the hole transport layer without being recombined in the light emitting layer, which may also be referred to as an electron inhibition layer. The electron blocking layer is preferably a material having a smaller electron affinity than the electron transport layer.

The light emitting material is preferably a material which may receive holes and electrons transported from a hole transport layer and an electron transport layer, respectively, and combine the holes and the electrons to emit light in a visible ray region, and has good quantum efficiency to fluorescence or phosphorescence. Specific examples of the light emitting material include an 8-hydroxy-quinoline aluminum complex (Alqs); a carbazole-based compound; a dimerized styryl compound; BAlq; a 10-hydroxybenzoquinoline-metal compound; a benzoxazole, benzthiazole and benzimidazole-based compound; a poly(p-phenylenevinylene)(PPV)-based polymer; a spiro compound; polyfluorene, rubrene, and the like, but are not limited thereto.

The light emitting layer may include a host material and a dopant material. The host material includes a fused aromatic ring derivative, a heterocycle-containing compound, or the like. Specific examples of the fused aromatic ring derivatives include anthracene derivatives, pyrene derivatives, naphthalene derivatives, pentacene derivatives, phenanthrene compounds, fluoranthene compounds, and the like. Examples of the heterocyclic-containing compounds include carbazole derivatives, dibenzofuran derivatives, ladder-type furan compounds, pyrimidine derivatives, and the like, but are not limited thereto. In particular, the compound according to the present disclosure can be used as a host for the light emitting layer.

Examples of the dopant material include an aromatic amine derivative, a styrylamine compound, a boron complex, a fluoranthene compound, a metal complex, and the like. Specifically, the aromatic amine derivative is a substituted or unsubstituted fused aromatic ring derivative having an arylamino group, and examples thereof include pyrene, anthracene, chrysene, periflanthene and the like, which have an arylamino group. The styrylamine compound is a compound where at least one arylvinyl group is substituted in substituted or unsubstituted arylamine, in which one or two or more substituent groups selected from the group consisting of an aryl group, a silyl group, an alkyl group, a cycloalkyl group, and an arylamino group are substituted or unsubstituted. Specific examples thereof include styrylamine, styryldiamine, styryltriamine, styryltetramine, and the like, but are not limited thereto. Further, the metal complex includes an iridium complex, a platinum complex, and the like, but is not limited thereto.

The electron transport layer is a layer which receives electrons from an electron injection layer and transports the electrons to a light emitting layer, and an electron transport material is suitably a material which may receive electrons well from a cathode and transfer the electrons to a light emitting layer, and has a large mobility for electrons. Specific examples of the electron transport material include: an Al complex of 8-hydroxyquinoline; a complex including Alqs; an organic radical compound; a hydroxyflavone-metal complex, and the like, but are not limited thereto. The electron transport layer may be used with any desired cathode material, as used according to the related art. In particular, appropriate examples of the cathode material are a typical material which has a low work function, followed by an aluminum layer or a silver layer. Specific examples thereof include cesium, barium, calcium, ytterbium, and samarium, in each case followed by an aluminum layer or a silver layer.

The electron injection layer is a layer which injects electrons from an electrode, and is preferably a compound which has a capability of transporting electrons, has an effect of injecting electrons from a cathode and an excellent effect of injecting electrons into a light emitting layer or a light emitting material, prevents excitons produced from the light emitting layer from moving to a hole injection layer, and is also excellent in the ability to form a thin film. Specific examples of the electron injection layer include fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like, but are not limited thereto.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

On the other hand, in the present disclosure, the "electron injection and transport layer" is a layer that performs both the roles of the electron injection layer and the electron transport layer, and the materials that perform the roles of each layer may be used alone or in combination, without being limited thereto.

The organic light emitting device according to the present disclosure may be a bottom emission device, a top emission device, or a double-sided light emitting device, and particularly, may be a bottom emission device that requires relatively high luminous efficiency.

In addition, the compound represented by Chemical Formula 1 can be included in an organic solar cell or an organic transistor in addition to an organic light emitting device.

Below, the embodiments are described in more detail to assist in the understanding of the present disclosure. However, the following examples are provided for illustrative purposes only, and are not intended to limit the subject matter of the present disclosure.

### [PREPARATIONEXAMPLE]

### Preparation Example 1 : Synthesis of Compound 1

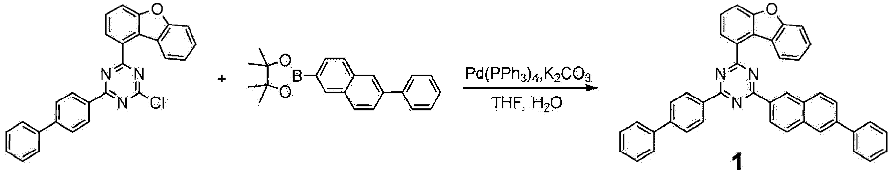

2-([1,1'-biphenyl]-4-yl)-4-chloro-6-(dibenzo[b,d]furan-1-yl)-1,3,5-triazine (15.0 g, 34.6 mmol) and 4,4,5,5-tetramethyl-2-(6-phenylnaphthalen-2-yl)-1,3,2-dioxaborolane(12.6 g, 38.0 mmol) were added to 300 ml of THF under a nitrogen atmosphere, and the mixture was stirred and refluxed. Then, potassium carbonate (19.1 g, 138.3 mmol) was dissolved in 57 ml of water and added thereto, and the mixture was sufficiently stirred and then tetrakis(triphenylphosphine)palladium(0) (0.1 g, 1.0 mmol) was added. After the reaction for 10 hours, the reaction mixture was cooled to room temperature, the organic layer and the aqueous layer were separated, and then the organic layer was distilled. This was dissolved again in chloroform, washed twice with water, and the organic layer was separated. Anhydrous magnesium sulfate was added, and the mixture was stirred, filtered, and the filtrate was distilled under reduced pressure. The concentrated compound was purified by silica gel column chromatography and then subjected to sublimation purification to prepare 7.5 g of Compound 1. (Yield: 36%, MS[M+H]⁺= 603)

### Preparation Example 2: Synthesis of Compound 2

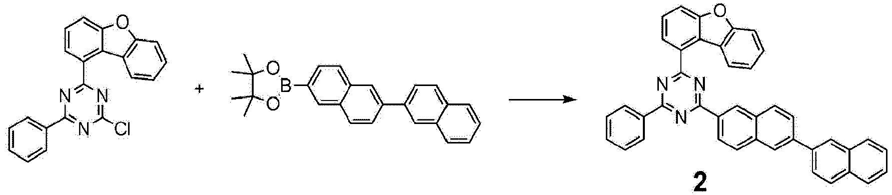

Compound 2 was prepared in the same manner as in the preparation method of Compound 1, except that in Preparation Example 1, 2-([1,1'-biphenyl]-4-yl)-4-chloro-6-(dibenzo[b,d]furan-1-yl)-1,3,5-triazine was changed to 2-chloro-4-(dibenzo[b,d]furan-1-yl)-6-phenyl-1,3,5-triazine, and 4,4,5,5-tetramethyl-2-(6-phenylnaphthalen-2-yl)-1,3,2-dioxaborolane was changed to 2-([2,2'-binaphthalen]-6-yl)-4,4,5,5-tetramethyl-1,3,2-dioxaborolane. (MS: [M+H]+ = 577)

### Preparation Example 3: Synthesis of Compound 3

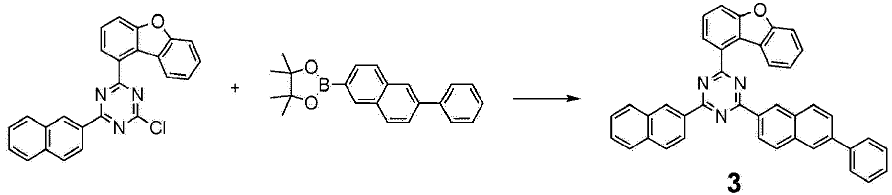

Compound 3 was prepared in the same manner as in the preparation method of Compound 1, except that in Preparation Example 1,2-([1,1'-biphenyl]-4-yl)-4-chloro-6-(dibenzo[b,d]furan-1-yl)-1,3,5-triazine was changed to 2-chloro-4-(dibenzo[b,d]furan-1-yl)-6-(naphthalen-2-yl)-1,3,5-triazine. (MS: [M+H]+ = 577)

### Preparation Example 4: Synthesis of Compound 4

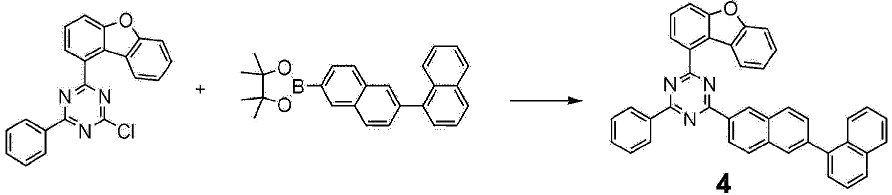

Compound 4 was prepared in the same manner as in the preparation method of Compound 1, except that in Preparation Example 1, 2-([1,1'-biphenyl]-4-yl)-4-chloro-6-(dibenzo[b,d]furan-1-yl)-1,3,5-triazine was changed to 2-chloro-4-(dibenzo[b,d]furan-1-yl)-6-phenyl-1,3,5-triazine, and 4,4,5,5-tetramethyl-2-(6-phenylnaphthalen-2-yl)-1,3,2-dioxaborolane was changed to 2-([1,2'-binaphthalen]-6'-yl)-4,4,5,5-tetramethyl-1,3,2-dioxaborolane. (MS: [M+H]+ = 577)

### Preparation Example 5: Synthesis of Compound 5

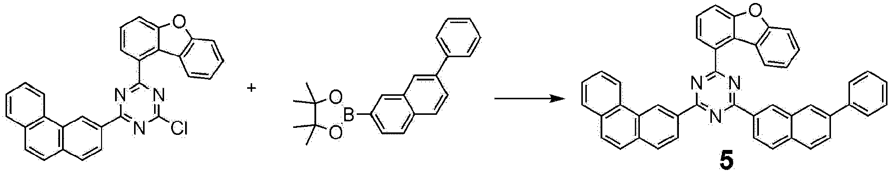

Compound 5 was prepared in the same manner as in the preparation method of Compound 1, except that in Preparation Example 1, 2-([1,1'-biphenyl]-4-yl)-4-chloro-6-(dibenzo[b,d]furan-1-yl)-1,3,5-triazine was changed to 2-chloro-4-(dibenzo[b,d]furan-1-yl)-6-(phenanthren-3-yl)-1,3,5-triazine, and 4,4,5,5-tetramethyl-2-(6-phenylnaphthalen-2-yl)-1,3,2-dioxaborolane was changed to 4,4,5,5-tetramethyl-2-(7-phenylnaphthalen-2-yl)-1,3,2-dioxaborolane. (MS: [M+H]+ = 627)

### Preparation Example 6: Synthesis of Compound 6

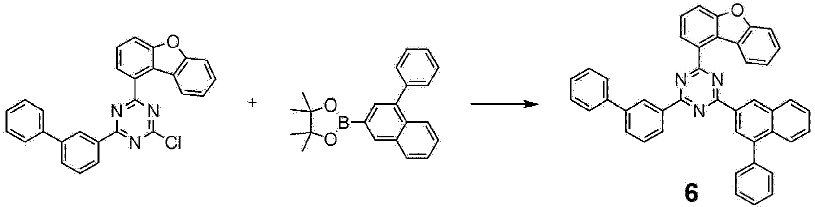

Compound 6 was prepared in the same manner as in the preparation method of Compound 1, except that in Preparation Example 1, 2-([1,1'-biphenyl]-4-yl)-4-chloro-6-(dibenzo[b,d]furan-1-yl)-1,3,5-triazine was changed to 2-([1,1'-biphenyl]-3-yl)-4-chloro-6-(dibenzo[b,d]furan-1-yl)-1,3,5-triazine, and 4,4,5,5-tetramethyl-2-(6-phenylnaphthalen-2-yl)-1,3,2-dioxaborolane was changed to 4,4,5,5-tetramethyl-2-(4-phenylnaphthalen-2-yl)-1,3,2-dioxaborolane. (MS: [M+H]+ = 603)

### Preparation Example 7: Synthesis of Compound 7

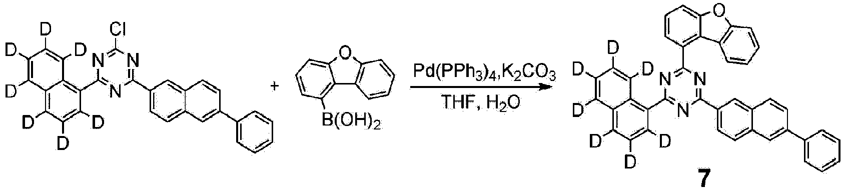

2-Chloro-4-(naphthalen-1-yl-d7)-6-(6-phenylnaphthalen-2-yl)-1,3,5-triazine (15 g, 33.3 mmol) and dibenzo[b,d]furan-1-ylboronic acid (7.8 g, 36.6 mmol) were added to 300 ml of THF under a nitrogen atmosphere, and the mixture was stirred and refluxed. Then, potassium carbonate (18.4 g, 133 mmol) was dissolved in 55 ml of water and added thereto, and the mixture was sufficiently stirred and then tetrakis(triphenylphosphine)palladium(0) (0.1 g, 1 mmol) was added. After the reaction for 9 hours, the reaction mixture was cooled to room temperature, the organic layer and the aqueous layer were separated, and then the organic layer was distilled. This was dissolved again in chloroform, washed twice with water, and the organic layer was separated. Anhydrous magnesium sulfate was added, and the mixture was stirred, filtered, and the filtrate was distilled under reduced pressure. The concentrated compound was purified by silica gel column chromatography to prepare 8.1 g of Compound 7. (Yield: 42%, MS[M+H]⁺= 584)

### Preparation Example 8: Synthesis of Compound 8

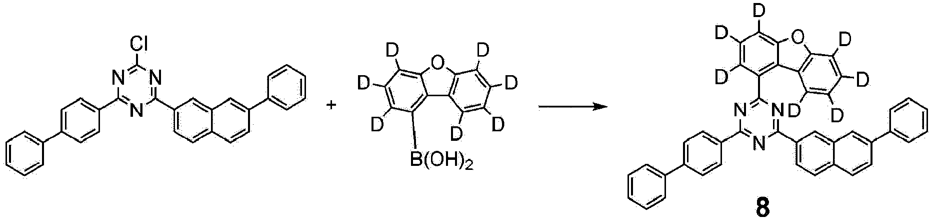

Compound 8 was prepared in the same manner as in the preparation method of Compound 7, except that in Preparation Example 7, 2-chloro-4-(naphthalen-1-yl-d7)-6-(6-phenylnaphtha len-2-yl)-1,3,5-triazine was changed to 2-([1,1'-biphenyl]-4-yl)-4-chloro-6-(7-phenylnaphthalen-2-yl)-1,3,5-triazine, and dibenzo[b,d]furan-1-ylboronic acid was changed to (dibenzo[b,d]furan-1-yl-d7)boronic acid. (MS: [M+H]+ = 610)

### [EXAMPLE]

### Comparative Example 1

A glass substrate on which ITO (indium tin oxide) was coated as a thin film to a thickness of 1,400 Å was put into distilled water in which a detergent was dissolved, and ultrasonically cleaned. At this time, Decon^{™} CON705 product manufactured by Fischer Co. was used as the detergent, and as the distilled water, distilled water filtered twice using a 0.22 µm sterilizing filter manufactured by Millipore Co. was used. After the ITO was cleaned for 30 minutes, ultrasonic cleaning was repeated twice using distilled water for 10 minutes. After the cleaning with distilled water was completed, the substrate was ultrasonically cleaned with solvents of isopropyl alcohol, acetone, and methanol for 10 minutes, and dried, after which it was transported to a plasma cleaner. Then, the substrate was cleaned with oxygen plasma for 5 minutes, and then transferred to a vacuum evaporator.

On the ITO transparent electrode thus prepared, the following Compound HI-A and Compound LG-101 were sequentially thermally vacuum-deposited to a thickness of 800 Å and 50 Å, respectively, to form a hole injection layer. The following Compound HT-A was vacuum-deposited thereon to a thickness of 800 Å as a hole transport layer, and then, the following Compound EB-A was thermally vacuum-deposited to a thickness of 600 Å as an electron blocking layer. Compound RH-A was applied as a host for the light emitting layer, and Compound RD-A was applied as a dopant, and the host and dopant were vacuum-deposited at a weight ratio of 98:2 to a thickness of 400 Å. Then, the following Compound ET-A and Compound Liq were thermally vacuum-deposited at a ratio of 1:1 to a thickness of 360 Å, and then Liq was vacuum-deposited to a thickness of 5 Å as an electron transport and injection layer.

Magnesium and silver were sequentially deposited in a weight ratio of 10: 1 to a thickness of 220 Å, and aluminum to a thickness of 1000 Å on the electron injection layer to form a cathode, thereby completing the manufacture of an organic light emitting device

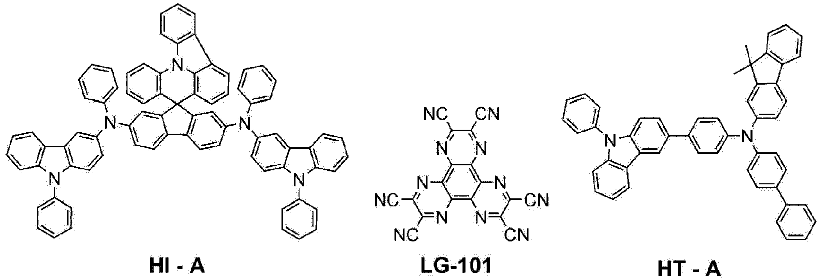

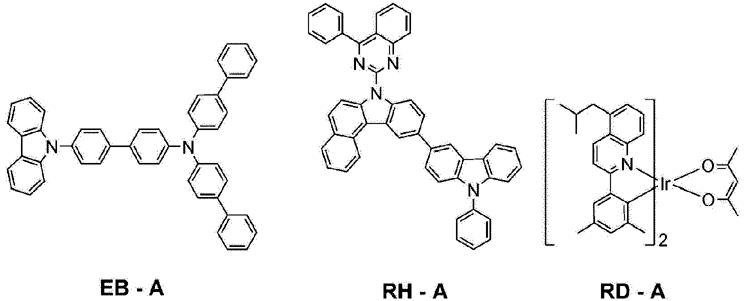

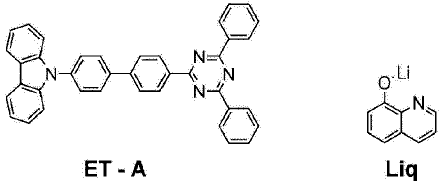

In the above-mentioned processes, the deposition rates of the organic materials were maintained at 0.4 ~0.7 Å/sec, the deposition rates of magnesium and silver were maintained at 2 Å/sec, and the degree of vacuum during the deposition was maintained at 2*10⁻⁷ ~ 5*10⁻⁶ torr, thereby manufacturing an organic light emitting device.

### Examples 1 to 16 and Comparative Examples 2 to 6

The organic light emitting devices of Examples 1 to 16 and Comparative Examples 2 to 6 were manufactured in the same manner as in Comparative Example 1, except that in Comparative Example 1, the light emitting layer host was changed as shown in Table 1 below instead of Compound 1. At this time, when a mixture of two types of Compounds was used as the host, the numbers inside the parentheses means the weight ratio between the host compounds. Compounds pRH-1, pRH-2, and Compounds A to C shown in Table 1 below are as follows.

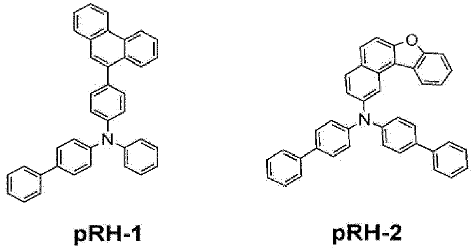

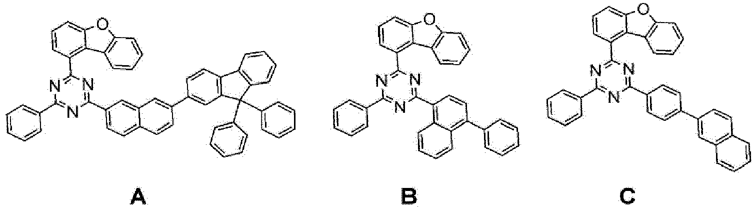

### [Experimental Example]

The driving voltage, efficiency and lifetime were measured by applying a current to the organic light emitting devices manufactured in Examples 1 to 16 and Comparative Examples 1 to 6, and the results are shown in Table 1 below. At this time, the voltage and efficiency were measured by applying a current density of 10 mA/cm², and LT97 means the time (hr) required for the luminance to be reduced to 97% of the initial luminance at a current density of 20 mA/cm².

**[Table 1]**

| | Light emitting layer host | @ 10 mA/cm² | | @ 20 mA/cm² |
|---|---|---|---|---|
| | | Driving voltage (V) | Efficiency (cd/A) | LT97(hr) |
| Example 1 | Compound 1:pRH-1 (50:50) | 4.43 | 22.8 | 124 |
| Example 2 | Compound 2:pRH-1 (50:50) | 4.40 | 22.2 | 121 |
| Example 3 | Compound 3:pRH-1 (50:50) | 4.44 | 21.9 | 119 |
| Example 4 | Compound 4:pRH-1 (50:50) | 4.46 | 20.9 | 110 |
| Example 5 | Compound 5:pRH-1 (50:50) | 4.39 | 21.3 | 107 |
| Example 6 | Compound 6:pRH-1 (50:50) | 4.36 | 22.2 | 113 |
| Example 7 | Compound 7:pRH-1 (50:50) | 4.46 | 21.8 | 173 |
| Example 8 | Compound 8:pRH-1 (50:50) | 4.40 | 20.4 | 176 |
| Example 9 | Compound 1:pRH-2 (50:50) | 4.33 | 21.3 | 121 |
| Example 10 | Compound 3:pRH-2 (50:50) | 4.35 | 21.8 | 110 |
| Example 11 | Compound 5:pRH-2 (50:50) | 4.42 | 21.7 | 106 |
| Example 12 | Compound 7:pRH-2 (50:50) | 4.38 | 21.6 | 164 |
| Example 13 | Compound 8:pRH-2 (50:50) | 4.39 | 21.0 | 172 |
| Example 14 | Compound 1 | 4.73 | 18.4 | 95 |
| Example 15 | Compound 3 | 4.75 | 18.1 | 86 |
| Example 16 | Compound 8 | 4.77 | 17.9 | 113 |
| Comparative Example 1 | Compound RH-A | 5.43 | 14.2 | 42 |
| Comparative Example 2 | Compound A:pRH-1 (50:50) | 5.62 | 17.3 | 52 |
| Comparative Example 3 | Compound B:pRH-1 (50:50) | 5.98 | 12.6 | 67 |
| Comparative Example 4 | Compound C:pRH-2 (50:50) | 5.23 | 16.7 | 63 |
| Comparative Example 5 | Compound A | 5.96 | 10.6 | 21 |
| Comparative Example 6 | Compound B | 6.23 | 8.4 | 23 |

From the results of Table 1 above, it was confirmed that when the compounds having the structure of Chemical Formula 1 were applied to the light emitting layer of an organic electroluminescent device, a device having low voltage, high efficiency, and long lifetime characteristics can be obtained.

### [Description of symbols]

| | | | |
|---|---|---|---|
| 1: | substrate | 2: | anode |
| 3: | light em itting layer | 4: | cathode |
| 5: | hole injection layer | 6: | hole transport layer |
| 7: | electron blocking layer transport layer | 8: | electron injection and |

## Claims

1. A compound represented by the following Chemical Formula 1: 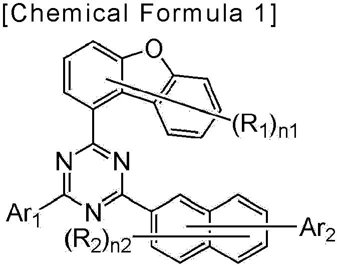 in Chemical Formula 1,
Ar₁ and Ar₂ are each independently a substituted or unsubstituted C₆₋₁₅ aryl,
R₁ and R₂ are each independently hydrogen or deuterium,
n1 is an integer of 1 to 7, and
n2 is an integer of 1 to 6.

2. The compound of claim 1, wherein:
the Chemical Formula 1 is represented by any one of the following Chemical Formula 1-1 to Chemical Formula 1-6 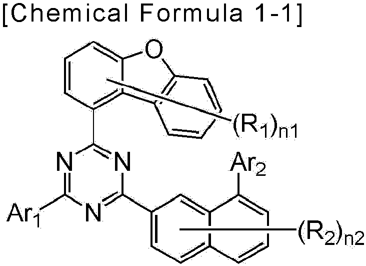 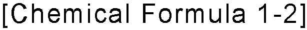 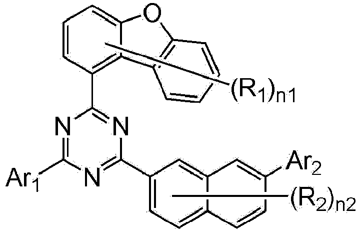 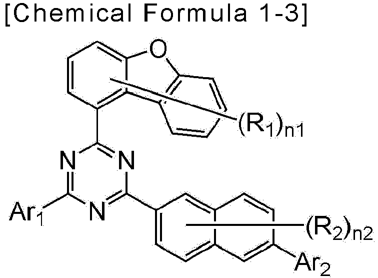 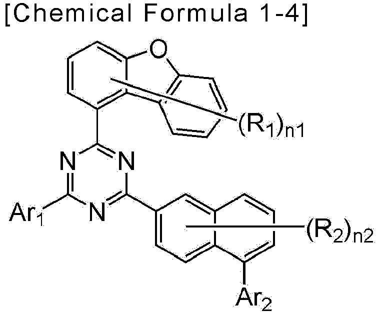 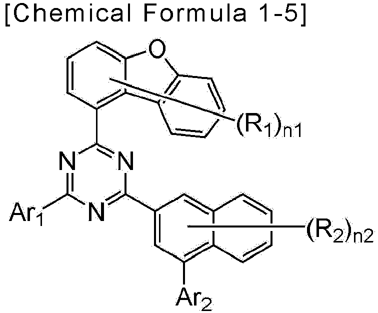 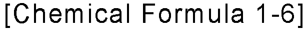 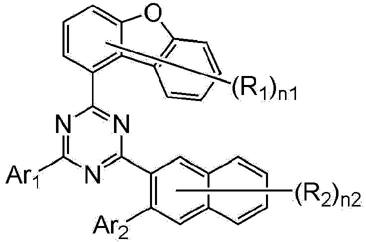 in Chemical Formula 1-1 to Chemical Formula 1-6, Ar₁, Ar₂, R₁, R₂, n1 and n2 are as defined in claim 1.

3. The compound of claim 1, wherein:
Ar₁ and Ar₂ are each independently phenyl, phenyl substituted with 3 or 5 deuteriums, biphenylyl, biphenylyl substituted with 5 or 9 deuteriums, naphthyl, naphthyl substituted with 7 deuteriums, phenanthrenyl, or phenanthrenyl substituted with 9 deuteriums.

4. The compound of claim 1, wherein:
Ar₁ and Ar₂ are each independently any one selected from the group consisting of:

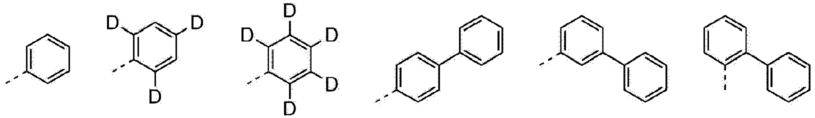

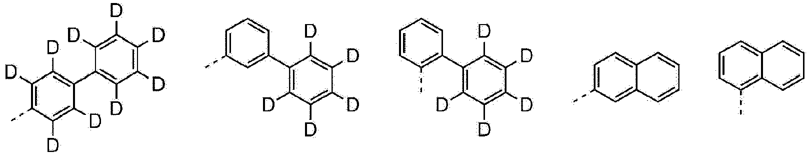

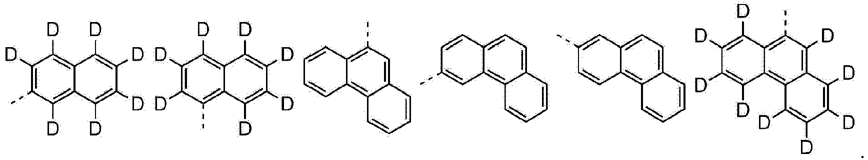

5. The compound of claim 1, wherein:
at least one of Ar₁ and Ar₂ is phenyl, or phenyl substituted with 3 or 5 deuteriums.

6. The compound of claim 1, wherein:
the compound represented by Chemical Formula 1 is any one selected from the group consisting of:

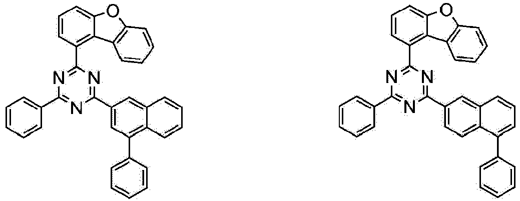

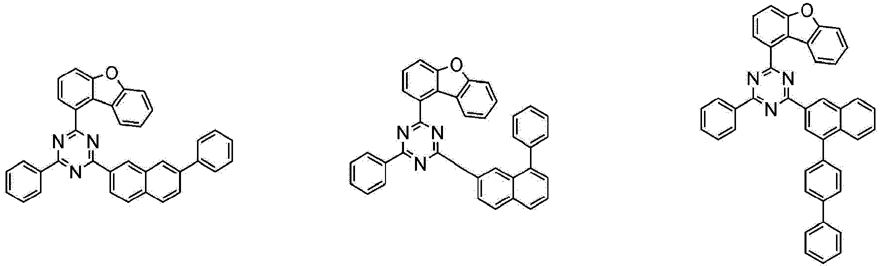

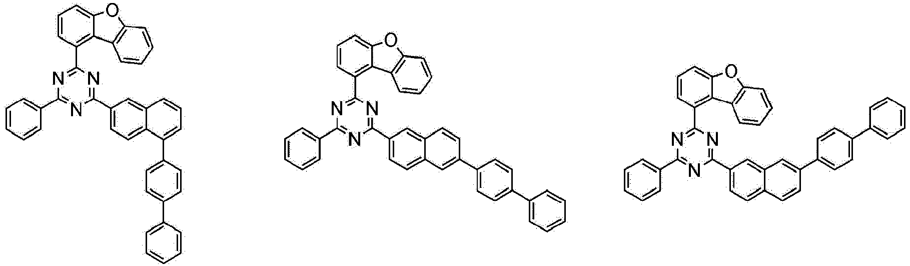

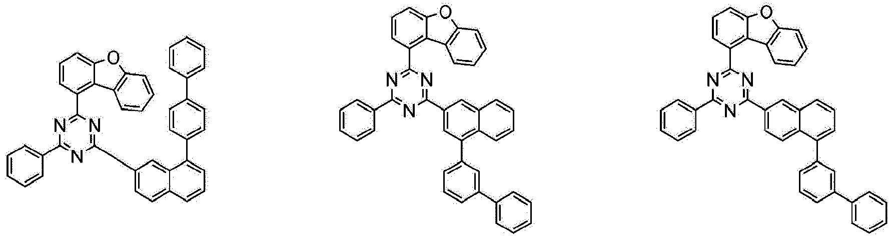

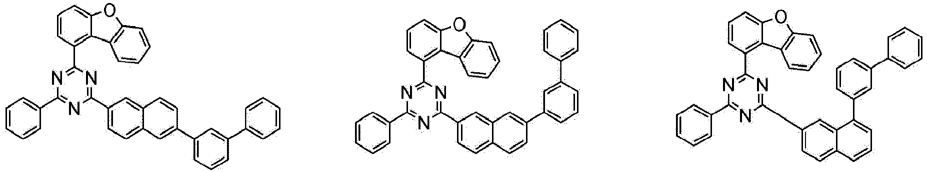

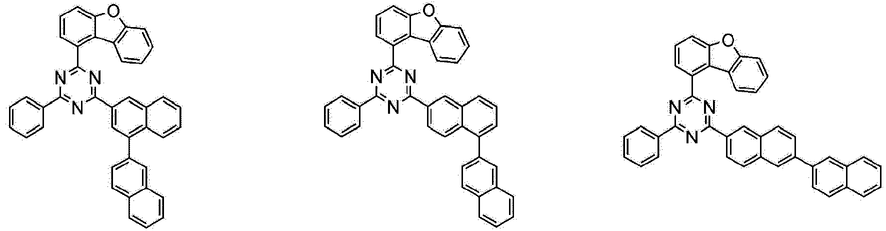

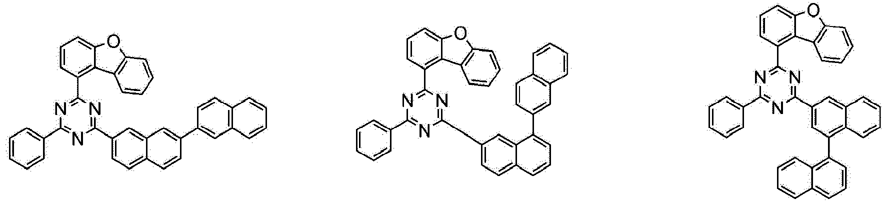

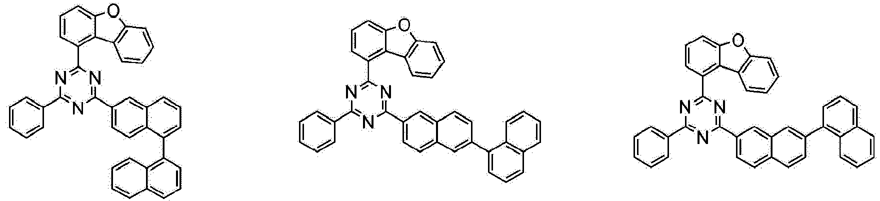

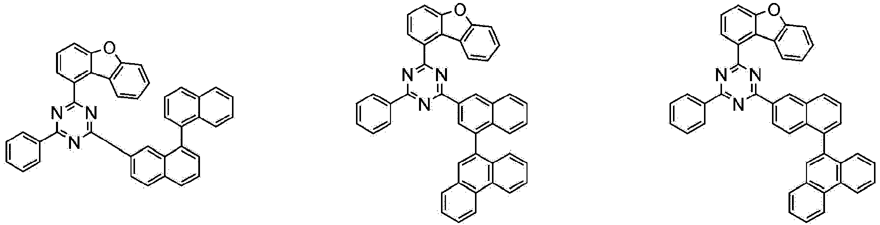

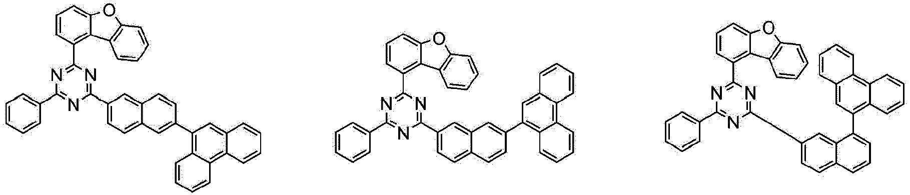

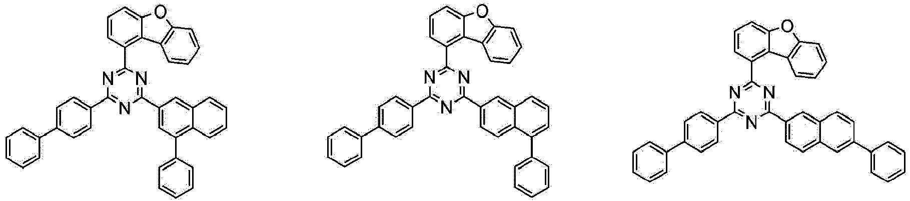

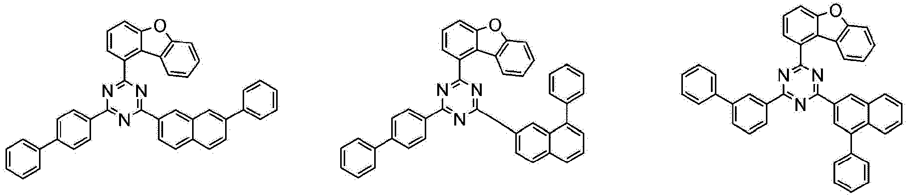

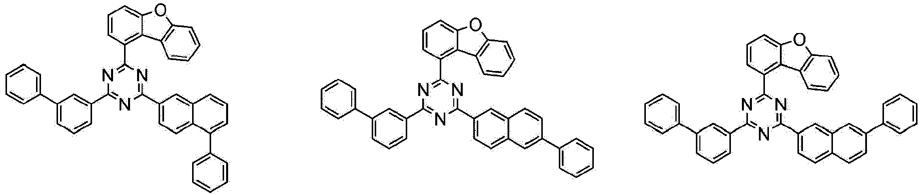

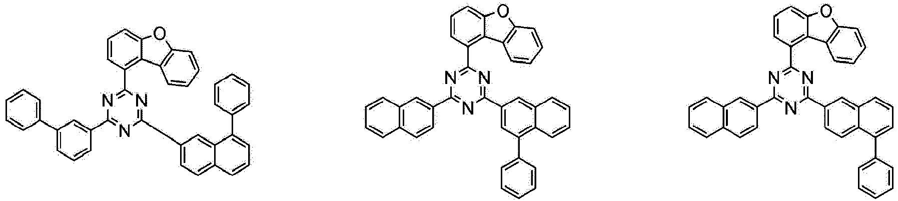

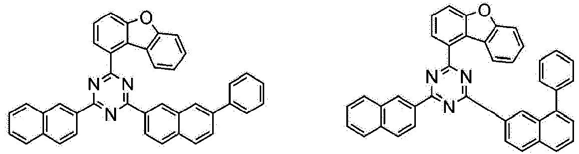

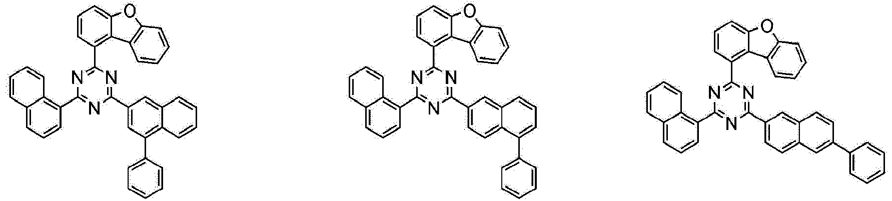

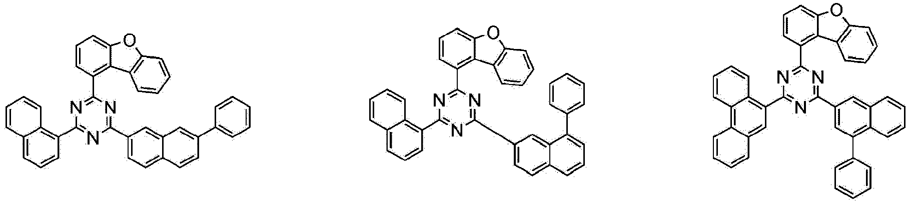

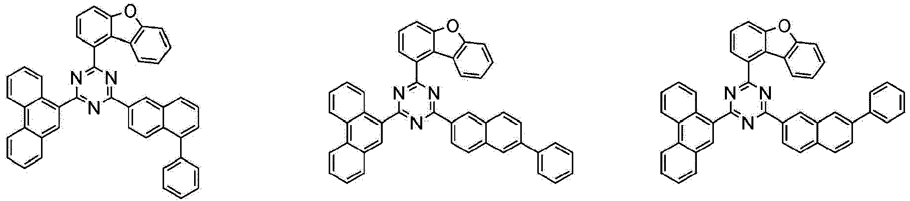

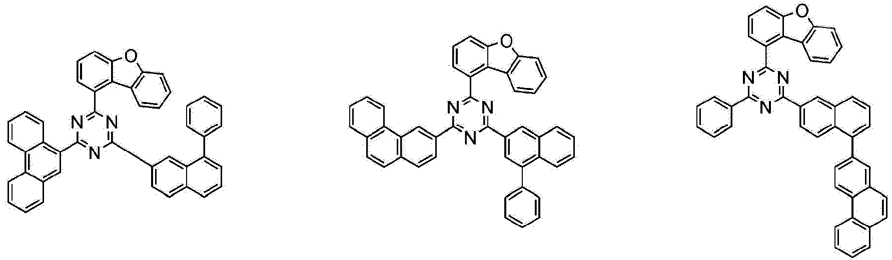

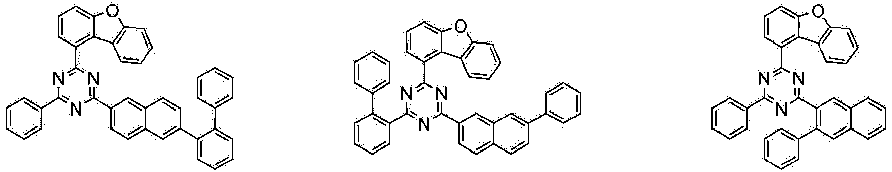

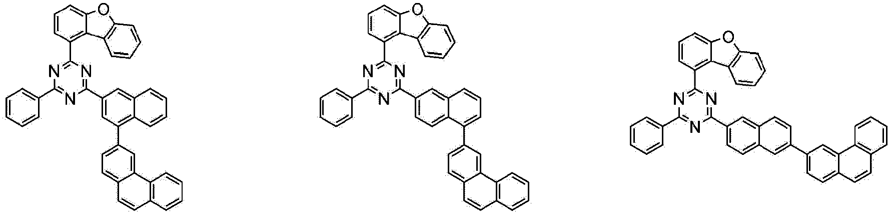

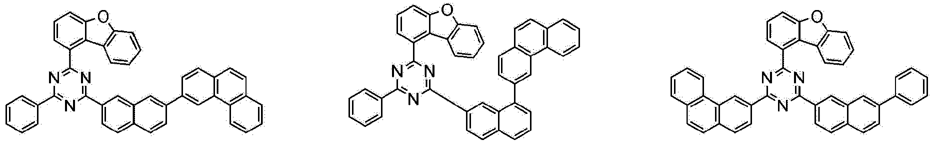

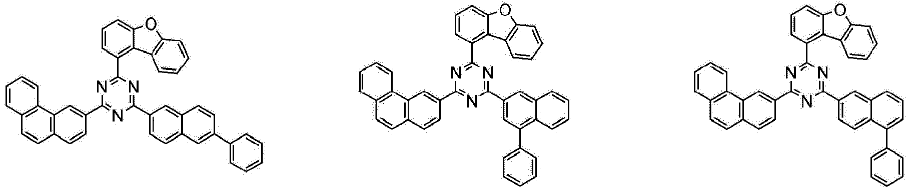

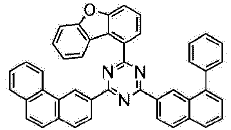

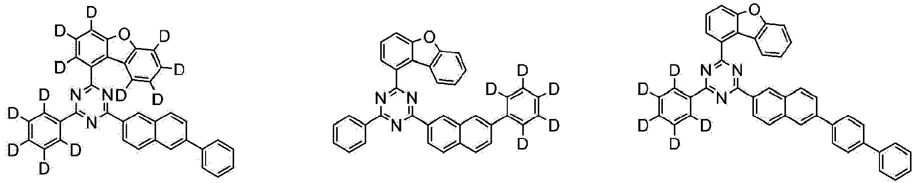

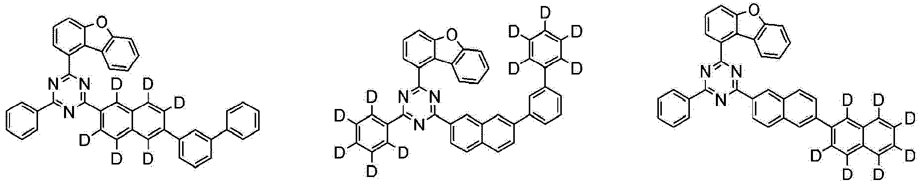

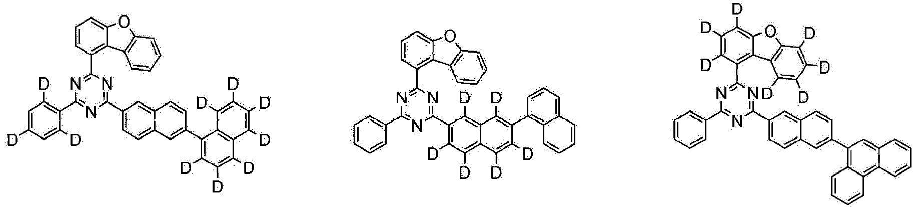

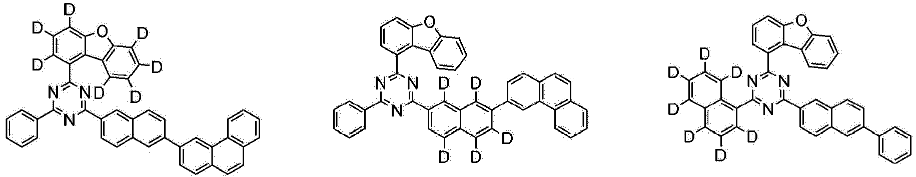

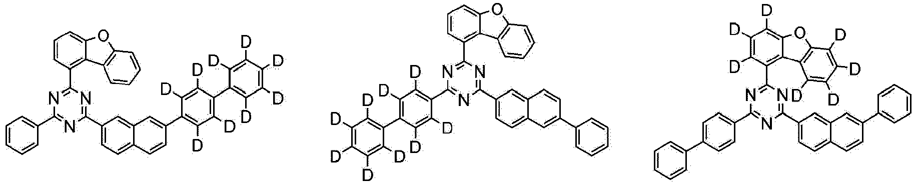

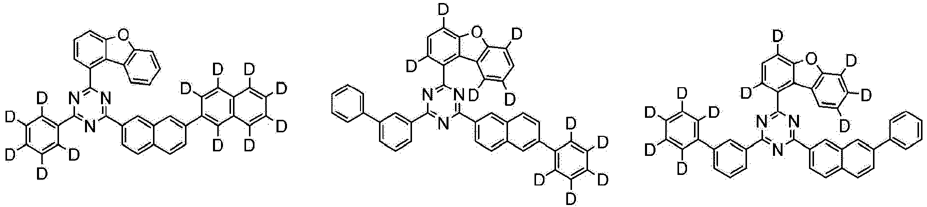

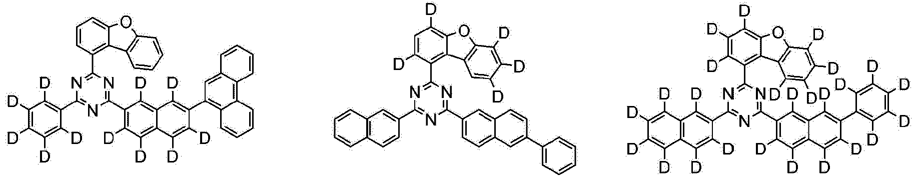

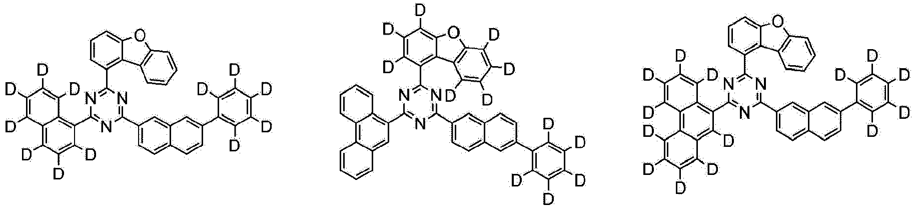

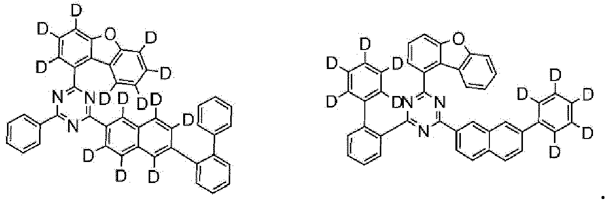

7. An organic light emitting device comprising: a first electrode; a second electrode that is provided opposite to the first electrode; and one or more organic material layers that are provided between the first electrode and the second electrode, wherein the one or more organic material layers comprise the compound as set forth in any one of claims 1 to 6.

8. The organic light emitting device of claim 7, wherein:
the organic material layer is a light emitting layer.
